# EUROPEAN PATENT SPECIFICATION

(11) **EP 2 853 280 B1**
(45) Date of publication and mention of the grant of the patent: **01.05.2019**
(21) Application number: 14173720.5
(22) Date of filing: 24.06.2014
(51) Int. Cl.: A61M 25/00, A61M 25/09

(54) **Guidewire**
Führungsdraht
Fil guide

(30) Priority: 25.09.2013 JP 2013197687
(43) Date of publication of application: 01.04.2015
(73) Proprietor: Asahi Intecc Co., Ltd., Seto-shi, Aichi 489-0071 (JP)
(72) Inventor: Takada, Keigo, Nagoya-shi, Aichi 463-0024 (JP)
(74) Representative: Winter, Brandl, Fürniss, Hübner, Röss, Kaiser, Polte - Partnerschaft mbB

(56) References cited:
- EP-A1- 1 875 941
- WO-A1-2004/007014
- US-A1- 2005 096 567
- US-A1- 2006 047 224
- US-A1- 2008 183 182

## Description

### Technical Field

The present invention relates to a guidewire inserted into the lumen of a blood vessel or the like.

### Background Art

A guidewire used when inserting a catheter into a blood vessel is known. When one wants to insert a catheter, the guidewire is first inserted into a blood vessel and the catheter advances along the guidewire. In this manner, the guidewire functions as a guide for guiding the catheter to a lesion.

As an example of such a guidewire, a guidewire (so-called a coil-type guidewire) in which a distal end of a core shaft is covered with a coil body is generally used. Moreover, a guidewire in which the surface of a coil body is covered with a coating such as a resin for the purpose of securing the ability to slide within the blood vessel is proposed (see US Patent No. 5,840,046 and Japanese Unexamined Patent Application No. JP 2008-237621 A).

US Patent No. 2006/0047224 A1 discloses a guidewire with a coating portion which covers a helical coil body. The coating portion comprises a first polymer having high conformance properties applied to a distal portion of the coil body wherein an outer surface of the first polymer substantially assumes a rippled shape of the outer surface of the underlying coil body structure, and a second polymer having relatively low conformance properties applied to a portion of the coil body proximal to the distal portion wherein an outer surface of the second polymer does not assume the rippled shape of the underlying coil body structure so as to be substantially smooth. In one embodiment of the guidewire disclosed in US Patent No. 2006/0047224 A1 the first and second polymers overlap one another such that the proximal end of the first polymer is proximal to the distal end of the second polymer.

However, in the conventional guidewire described above, since the surface of the coil body is covered with a coating, there is a problem that the coil body (and the guidewire) is rarely curved.

### Summary of the Invention

### Technical Problem

The present invention has been made to solve the above-described problems of the conventional art and aims to provide a technique for enabling a coil body to be easily curved in a guidewire in which the surface of the coil body is covered with a coating.

### Solution to Problem

In order to solve the above-described problems, a guidewire of the present invention employs the following configuration. That is, the guidewire includes a core shaft; a coil body that covers the core shaft; and a coating portion that covers the coil body, in which the coating portion is formed by stacking a plurality of films, a lowermost layer film of the coating portion is arranged to come between wires of the coil body, and a void is formed between a portion in which the lowermost layer film is arranged to come between the wires of the coil body and a film disposed above the lowermost layer film.

In such a guidewire of the present invention, the coating portion is formed by stacking a plurality of films, the lowermost layer film of the coating portion is arranged to come between the wires of the coil body, and a void is formed between a portion in which the lowermost layer film is arranged to come between the wires of the coil body and a film disposed above the lowermost layer film. Thus, it is possible to make the coating portion easily bent and to improve flexibility of the coating portion. As a result, it is possible to provide the guidewire in which the coil body is covered with the coating portion and the coil body (and the guidewire) can be easily curved.

Moreover, in the guidewire of the present invention, the coating portion may be formed by stacking at least three layer films, and a void may be formed between respective films of the coating portion in a portion in which the lowermost layer film of the coating portion is arranged to come between the wires of the coil body.

In such a guidewire of the present invention, the coating portion is formed by stacking at least three layer films, and a void is formed between respective films of the coating portion in a portion in which the lowermost layer film of the coating portion is arranged to come between the wires of the coil body. Thus, it is possible to improve the bendability and flexibility of the coating portion and to improve the strength of the coating portion. As a result, it is possible to provide a guidewire in which the coil body can be easily curved and the coating portion is rarely worn out.

Moreover, in the guidewire of the present invention, the coating portion may be formed such that an upper layer film is more flexible than a lower layer film.

When the guidewire (and the coil body) is curved, an upper layer film of the coating portion is deformed more than a lower layer film. Thus, as in the guidewire of the present invention, since the upper layer film of the coating portion is more flexible than the lower layer film, the coil body can be curved more easily. As a result, for example, even when a guidewire is inserted into a blood vessel that meanders complexly, it is possible to provide a guidewire that can follow the blood vessel satisfactorily.

The embodiments and/or examples of the following description which are not covered by the appended claims are considered as not being part of the present invention. The invention is defined in the appended set of claims.

### Brief Description of Drawings

FIG. 1 is a diagram for describing a configuration of a guidewire according to a first embodiment of the present invention.
FIG. 2 is an enlarged view of a coil body and a coating portion of the guidewire according to the first embodiment of the present invention.
FIG. 3 is an enlarged view of a coil body and a coating portion of a guidewire according to a second embodiment of the present invention.
FIG. 4 is an enlarged view of a coil body and a coating portion of a guidewire according to a third embodiment of the present invention.
FIG. 5 is an enlarged view of a coil body and a coating portion of a guidewire according to a fourth embodiment of the present invention.

### Description of Embodiments

### A. First Embodiment

Hereinafter, various embodiments of a guidewire of the present invention will be described in order to clarify the contents of the present invention.

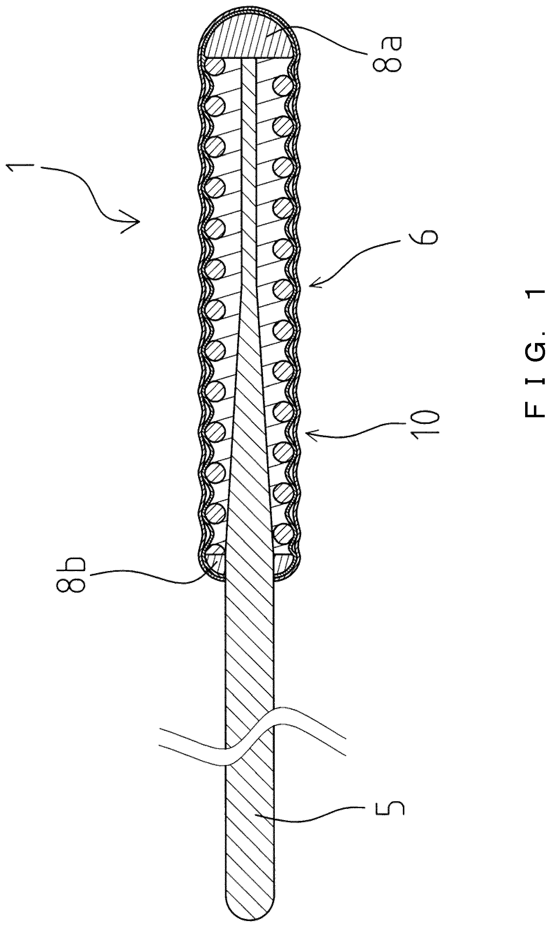

FIG. 1 is a diagram for describing a configuration of a guidewire 1 according to a first embodiment of the present invention. The guidewire 1 includes a core shaft 5, a coil body 6 that covers the core shaft 5, and the like. The core shaft 5 and the coil body 6 are joined together by a junction portion formed of a solder material or the like. In the present embodiment, a distal end of the coil body 6 and a distal end of the core shaft 5 are connected by a junction portion 8a, and a base end of the coil body 6 and an intermediate portion of the core shaft 5 are connected by a junction portion 8b.

Moreover, in the guidewire 1 of the present embodiment, the surface of the coil body 6 is covered with a coating portion 10. The coating portion 10 is provided to reduce frictional resistance between the surface of the guidewire 1 and the inner wall of a blood vessel to secure slidability when the guidewire 1 is inserted into the blood vessel. Thus, the coating portion 10 is preferably formed of a material (a hydrophilic resin or the like) having small frictional resistance. For example, the coating portion 10 is preferably formed of polyvinyl alcohol, polyvinylpyrrolidone, polyethylene glycol, polyacrylamide, a polyacrylic acid, sodium polyacrylate, poly(2-hydroxyethyl methacrylate), a maleic anhydride-based copolymer, an ethylene vinyl alcohol copolymer, 2-methacryloyloxyethyl phosphorylcholine or the copolymer, a (2-hydroxyethyl methacrylate)-styrene block copolymer, various synthetic polypeptides, collagen, hyaluronic acid, a cellulose-based polymer and mixtures thereof.

Moreover, the coating portion 10 may be formed by adding an additive such as a non-hydrophilic monomer, a cross-linker, a non-volatile solvent, a volatile solvent, or a surfactant to the above-described material.

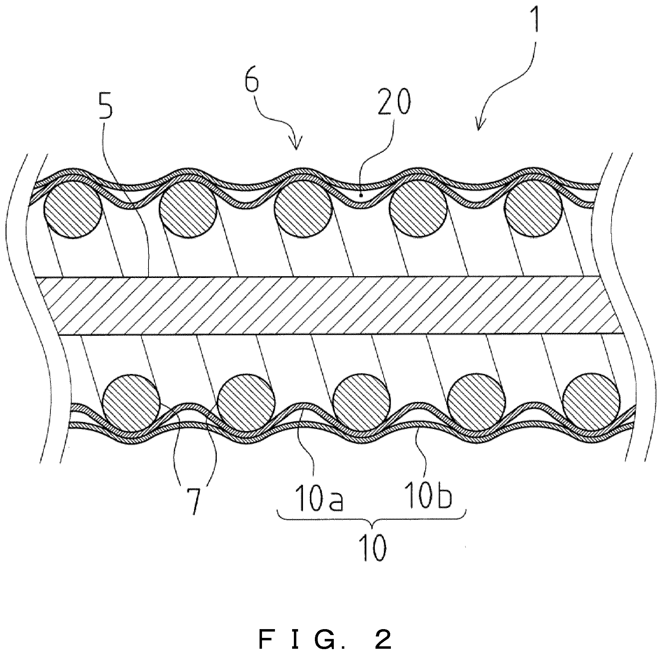

FIG. 2 is an enlarged view of the coil body 6 and the coating portion 10 of the guidewire 1 according to the first embodiment of the present invention. As illustrated in the drawing, the coil body 6 of the guidewire 1 according to the present embodiment is formed in such a shape (so-called a loose winding) that adjacent wires 7 are not in contact with each other.

Moreover, the coating portion 10 on the surface of the coil body 6 is formed by stacking a plurality of films (in the present embodiment, a lower layer film 10a and a upper layer film 10b).

Here, in the guidewire 1 of the present embodiment, the lower layer film 10a of the coating portion 10 is arranged to come between the wires of the coil body 6, and a void 20 is formed between a portion in which the lower layer film 10a is arranged to come between the wires of the coil body 6 and the upper layer film 10b.

In such a guidewire 1 of the present embodiment, since the void 20 is formed between the portion in which the lower layer film 10a is arranged to come between the wires of the coil body 6 and the upper layer film 10b, the coating portion 10 is easily bent and the coating portion 10 is sufficiently flexible.

Here, in general, when the surface of the coil body is covered with the coating portion, the coating portion supports when the coil body is curved whereby the coil body is rarely curved. As a result, the ability of the guidewire to follow the blood vessel tends to decrease. On the other hand, when the surface of the coil body is not covered with the coating portion, it may be difficult to secure the ability of the guidewire to slide within the blood vessel.

Due to the above reasons, in the related art, it was difficult to secure the abilities of the guidewire to slide within the blood vessel and follow the blood vessel.

In contrast, in the guidewire 1 of the present embodiment, since the coating portion 10 is easily bent and has excellent flexibility, it is possible to easily bend the coil body 6 while having a configuration in which the coating portion 10 is present on the surface of the coil body 6. As a result, it is possible to secure the abilities of the guidewire 1 to slide within the blood vessel and follow the blood vessel.

The abovementioned first embodiment has other associated embodiments. Hereinafter, the other embodiments will be described briefly. In the following description, the same configurations as those of the guidewire 1 of the first embodiment will be denoted by the same reference numerals, and detailed description thereof will not be provided.

### B. Second Embodiment

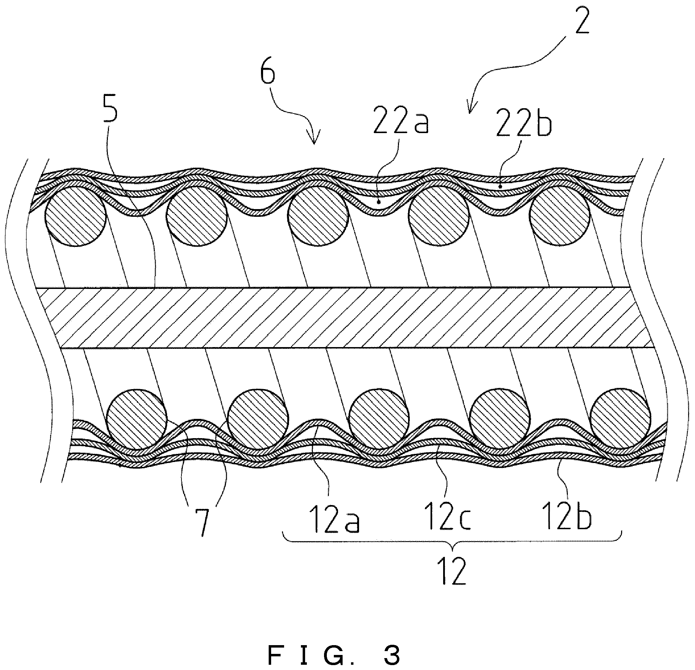

FIG. 3 is an enlarged view of the coil body 6 and the coating portion 12 of a guidewire 2 according to a second embodiment. The guidewire 2 of the present embodiment has the following differences from the guidewire 1 of the first embodiment. That is, as illustrated in FIG. 3, a coating portion 12 that covers the coil body 6 includes three layer films (a lower layer film 12a, an intermediate layer film 12c, and an upper layer film 12b). Moreover, a void 22a is formed between a portion in which the lower layer film 12a is arranged to come between the wires 7 of the coil body 6 and the intermediate layer film 10c, and a void 22b is formed between the intermediate layer film 12c and the upper layer film 12b.

In the guidewire 2 of the present embodiment, the core shaft 5 is inserted into the lumen of the coil body 6 similarly to the guidewire 1 of the first embodiment.

In such a guidewire 2 of the present embodiment, since the number of films constituting the coating portion 12 is larger than that of the guidewire 1 of the first embodiment, it is possible to improve the strength of the coating portion 12.

Moreover, since the void 22a is formed between the lower layer film 12a and the intermediate layer film 12b and the void 22b is formed between the intermediate layer film 12c and the upper layer film 12b, it is possible to sufficiently secure the bendability and the flexibility even when the number of films of the coating portion 12 is increased.

As described above, according to the guidewire 2 of the present embodiment, since the strength of the coating portion 12 can be improved while securing the bendability and the flexibility of the coating portion 12, it is possible to provide the guidewire 2 in which the coating portion 12 is rarely broken and the coil body 6 is easily bendable.

### C. Third Embodiment

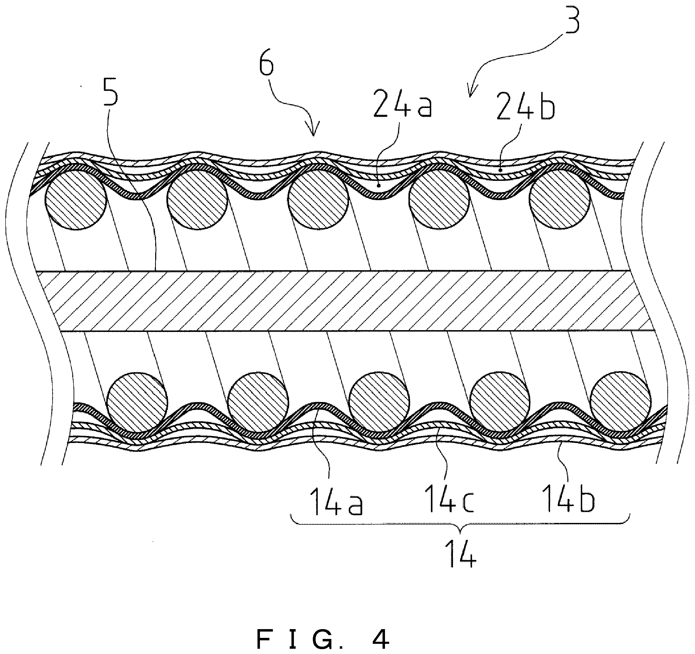

FIG. 4 is an enlarged view of the coil body 6 and the coating portion 14 of a guidewire 3 according to a third embodiment. In FIG. 4, a difference in hatching density of the films (a lower layer film 14a, an intermediate layer film 14c, and an upper layer film 14b) constituting the coating portion 14 indicates a difference in the flexibility of respective films. That is, a lightly hatched film has higher flexibility than a densely hatched film.

As illustrated in FIG. 4, in the present embodiment, the intermediate layer film 14c is more flexible than the lower layer film 14a, and the upper layer film 14b is more flexible than the intermediate layer film 14c.

In the present embodiment, the lower layer film 14a, the intermediate layer film 14c, and the upper layer film 14b are formed using different resins so that the respective films have different flexibility. However, the lower layer film 14a, the intermediate layer film 14c, and the upper layer film 14b may be formed using the same resin (for example, polyvinyl alcohol or the like) and the concentration of a cross-linker added to the resin may be adjusted so that the respective films have different flexibility. According to this method, it is possible to obtain desirable flexibility for a film easily.

Moreover, instead of forming the respective films of the coating portion 14 using different resins and adjusting the concentration of the cross-linkers of the respective films, the respective films may have different thicknesses (the intermediate layer film 14c may be thinner than the lower layer film 14a and the upper layer film 14b may be thinner than the intermediate layer film 14c). Above all, it is difficult to strictly control the thicknesses of the respective films of the coating portion 14. Thus, as described above, it becomes easy to manufacture the guidewire 3 when the respective films are made to have different flexibility by using different resins and adjusting the concentration of cross-linkers.

The other configurations of the guidewire 3 of the present embodiment are the same as those of the guidewire 2. That is, the core shaft 5 is inserted into the lumen of the coil body 6. Moreover, a void 24a is formed between a portion in which the lower layer film 14a of the coating portion 14 is arranged to come between the wires 7 of the coil body 6, and a void 24b is formed between the intermediate layer film 14c and the upper layer film 14b.

In such a guidewire 3 of the present embodiment, similarly to the guidewire 1 of the first embodiment and the guidewire 2 of the second embodiment, a void (voids 24a and 24b) is formed between the plurality of films (the lower layer film 14a, the intermediate layer film 14c, and the upper layer film 14b) constituting the coating portion 14. Thus, it is possible to improve the bendability and flexibility of the coating portion 14 and make the coil body 6 (the guidewire 3) easily bent.

Moreover, when the coil body 6 is curved, an upper layer film of the coating portion 14 is deformed more than a lower layer film. Thus, as in the guidewire 3 of the present embodiment, by configuring the upper layer film that is deformed greatly when the coil body 6 is curved so as to be more flexible than the lower layer film, it is possible to make the coil body 6 curved more easily. As a result, it is possible to provide a guidewire having satisfactory followability even when the guidewire is inserted into a blood vessel that meanders complexly.

Although various embodiments of the guide wire have been described, the present invention is not limited to the embodiments. For example, in the guidewires of the second and third embodiments, a void is formed between the intermediate layer film and the upper layer film of the coating portion (see FIGS. 3 and 4). However, no void may be formed between the intermediate layer film and the upper layer film (the intermediate layer film may be closely attached to the upper layer film).

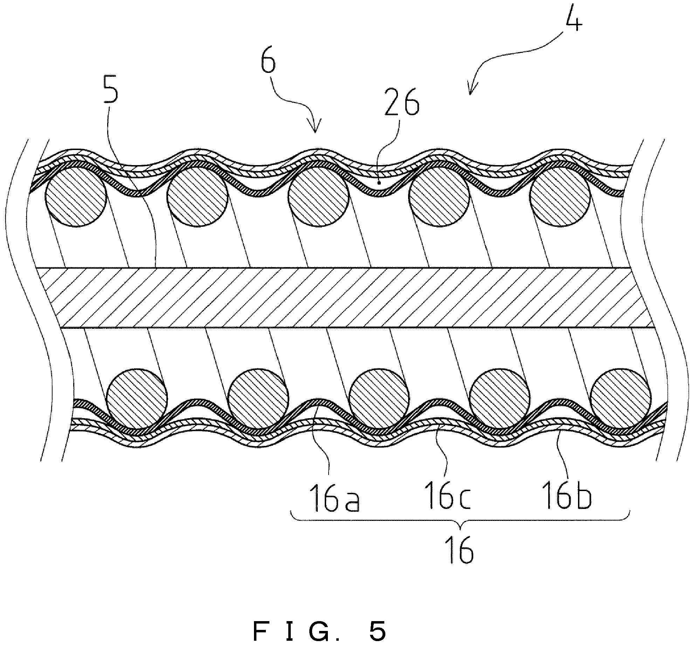

FIG. 5 is an enlarged view of a coil body 6 and a coating portion 16 of a guidewire 4 according to a fourth embodiment. As illustrated in the drawing, in the guidewire 4 of the present embodiment, although a void 26 is formed between a lower layer film 16a and an intermediate layer film 16c of the coating portion 16, the intermediate layer film 16c is closely attached to an upper layer film 16b and no void is formed between the films.

In the guidewire 4 of the present embodiment, the core shaft 5 is inserted into the lumen of the coil body 6 similarly to the guidewires of the above-described embodiments.

In such a guidewire 4 of the present embodiment, since the void 26 is formed between the lower layer film 16a and the intermediate layer film 16c of the coating portion 16, it is possible to improve the bendability and flexibility of the coating portion 16 and to make the coil body 6 easily bent.

Moreover, since the intermediate layer film 16c is closely attached to the upper layer film 16b, it is possible to make the upper layer film 16b rarely peel off from the intermediate layer film 16c.

Moreover, in the guidewires of the above-described embodiments, the coating portion includes two or three layer films (see FIGS. 2 to 5). However, the coating portion may include at least four layer films (not illustrated).

However, if the coating portion includes too many layer films, since it is disadvantageous from the perspective of the bendability and flexibility of the coating portion, as in the above-described embodiments, the coating portion preferably includes two or three layer films.

Moreover, in the guidewires of the above-described embodiments, the coil body is formed in such a shape (loose winding) that adjacent wires are not in contact with each other. However, the coil body may be formed in such a shape (dense winding) that adjacent wires are in contact with each other (not illustrated).

However, when the coil body is formed in a loose winding, it is possible to increase the size of the void. As a result, it is possible to sufficiently secure the bendability and flexibility of the coating portion. Thus, the coil body is preferably formed in a loose winding as in the guidewires of the above-described embodiments.

### Reference Signs List

1, 2, 3, 4: Guidewire
5: Core shaft
6: Coil body
7: wire
8a, 8b: Junction portion
10, 12, 14, 16: Coating portion
10a, 12a, 14a, 16a: Lower layer film
12c, 14c, 16c: Intermediate layer film
10b, 12b, 14b, 16b: Upper layer film
20, 22a, 22b, 24a, 24b, 26: Void

## Claims

1. A guidewire (1; 2; 3; 4) comprising:
a core shaft (5);
a coil body (6) that covers the core shaft (5); and
a coating portion (10; 12; 14; 16) that covers the coil body (16), wherein
the coating portion (10; 12; 14; 16) is formed by stacking a plurality of films (10a, 10b; 12a, 12b, 12c; 14a, 14b, 14c; 16a, 16b, 16c), and
a lowermost layer film (10a; 12a; 14a; 16a) of the coating portion (10; 12; 14; 16) is arranged to come between wires (7) of the coil body (6), **characterized in that**
a void (20; 22a; 24a; 26) is formed between a portion in which the lowermost layer film (10a; 12a; 14a; 16a) is arranged to come between the wires (7) of the coil body (6) and a film (10b, 12c; 14c; 16c) disposed above the lowermost layer film (10a; 12a; 14a; 16).

2. The guidewire (2; 3) according to claim 1, wherein
the coating portion (12; 14) is formed by stacking at least three layer films (12a, 12b, 12c; 14a, 14b, 14c), and
a void (22a, 22b; 24a, 24b) is formed between respective films of the coating portion (12, 14) in a portion in which the lowermost layer film (12a; 14a) of the coating portion (12; 14) is arranged to come between the wires (7) of the coil body (6).

3. The guidewire (3) according to claim 1 or 2, wherein
the coating portion (14) is formed such that an upper layer film (14b, 14c) is more flexible than a lower layer film (14a).

## Patentansprüche

1. Führungsdraht (1; 2; 3; 4) mit:
einem Kernschaft (5);
einem den Kernschaft (5) ummantelnden Wicklungskörper (6); und
einer den Wicklungskörper (16) überziehenden Beschichtung (10; 12; 14; 16), wobei
die Beschichtung (10; 12; 14; 16) aus einer Vielzahl übereinanderliegender Filme (10a, 10b; 12a, 12b, 12c; 14a, 14b, 14c; 16a, 16b, 16c) gebildet ist, und
ein unterstliegender Film (10a; 12a; 14a; 16a) der Beschichtung (10; 12; 14; 16) zwischen Drähten (7) des Wicklungskörpers (6) eingedrungen ist, **dadurch gekennzeichnet, dass**
zwischen einem Bereich, in dem der unterstliegende Film (10a; 12a; 14a; 16a) zwischen den Drähten (7) des Wicklungskörpers (6) eingedrungen ist, und einem über dem unterstliegenden Film (10a; 12a; 14a; 16) liegenden Film (10b, 12c; 14c; 16c) ein Hohlraum (20; 22a; 24a; 26) ausgebildet ist.

2. Führungsdraht (2; 3) nach Anspruch 1, wobei
die Beschichtung (12; 14) aus wenigstens drei übereinanderliegenden Filmen (12a, 12b, 12c; 14a, 14b, 14c) gebildet ist, und
zwischen jeweiligen Filmen der Beschichtung (12, 14) in einem Bereich, in dem der unterstliegende Film (12a; 14a) der Beschichtung (12; 14) zwischen den Drähten (7) des Wicklungskörpers (6) eingedrungen ist, ein Hohlraum (22a, 22b; 24a, 24b) ausgebildet ist.

3. Führungsdraht (3) nach Anspruch 1 oder 2, wobei
die Beschichtung (14) so ausgebildet ist, dass ein obenliegender Film (14b, 14c) flexibler ist als ein untenliegender Film (14a).

## Revendications

1. Fil-guide (1 ; 2 ; 3 ; 4) comprenant :
une tige centrale (5) ;
un corps hélicoïdal (6) qui recouvre la tige centrale (5) ; et
une partie de revêtement (10 ; 12 ; 14 ; 16) qui recouvre le corps hélicoïdal (16) ; dans lequel :
la partie de revêtement (10 ; 12 ; 14 ; 16) est formée en empilant une pluralité de films (10a, 10b ; 12a, 12b, 12c ; 14a, 14b, 14c ; 16a, 16b, 16c), et
un film formant couche la plus basse (10a ; 12a ; 14a ; 16a) de la partie de revêtement (10 ; 12 ; 14 ; 16) est agencé pour venir entre les fils (7) du corps hélicoïdal (6), **caractérisé en ce que** :
un vide (20 ; 22a ; 24a ; 26) est formé entre une partie dans laquelle le film formant couche la plus basse (10a ; 12a ; 14a ; 16a) est agencé pour venir entre les fils (7) du corps hélicoïdal (6) et un film (10b, 12c ; 14c ; 16c) disposé au-dessus du film formant couche de la plus basse (10a ; 12a ; 14a ; 16).

2. Fil-guide (2 ; 3) selon la revendication 1, dans lequel :
la partie de revêtement (12 ; 14) est formée en empilant au moins trois films formant couche (12a, 12b, 12c ; 14a, 14b, 14c), et
un vide (22a, 22b ; 24a, 24b) est formé entre des films respectifs de la partie de revêtement (12, 14) dans une partie dans laquelle le film formant couche la plus basse (12a ; 14a) de la partie de revêtement (12 ; 14) est agencé pour venir entre les fils (7) du corps hélicoïdal (6).

3. Fil-guide (3) selon la revendication 1 ou 2, dans lequel :
la partie de revêtement (14) est formée de sorte qu'un film formant couche supérieure (14b, 14c) est plus flexible qu'un film formant couche inférieure (14a).
